(19) Europäisches Patentamt
European Patent Office
Office européen des brevets

(11) **EP 2 762 061 B1**

(12) **EUROPÄISCHE PATENTSCHRIFT**

(45) Veröffentlichungstag und Bekanntmachung des Hinweises auf die Patenterteilung:
**27.05.2020 Patentblatt 2020/22**

(51) Int Cl.:
*A61B 5/00* (2006.01)  *A61B 5/053* (2006.01)
*A61B 5/08* (2006.01)

(21) Anmeldenummer: **14151412.5**

(22) Anmeldetag: **16.01.2014**

(54) **Elektroimpedanztomographie-Gerät und -Verfahren**

Electro-impedance tomography device and method

Appareil et procédé de tomographie par impédance électrique

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.02.2013 DE 102013201804**
**10.07.2013 DE 102013213526**

(43) Veröffentlichungstag der Anmeldung:
**06.08.2014 Patentblatt 2014/32**

(73) Patentinhaber: **Drägerwerk AG & Co. KGaA**
**23558 Lübeck (DE)**

(72) Erfinder:
• **Gärber, Yvo**
**23881 Breitenfelde (DE)**
• **Stenqvist, Ola**
**26040 Viken (SE)**

(74) Vertreter: **Uexküll & Stolberg**
**Partnerschaft von**
**Patent- und Rechtsanwälten mbB**
**Beselerstraße 4**
**22607 Hamburg (DE)**

(56) Entgegenhaltungen:
**EP-A1- 2 228 009**

• **K Lowhagen ET AL: "Regional intratidal gas distribution in acute lung injury and acute respiratory distress syndrome--assessed by electric impedance tomography", Minerva anestesiologica, 1. Dezember 2010 (2010-12-01), Seiten 1024-1035, XP055115067, Italy Gefunden im Internet:**
**URL:http://gup.ub.gu.se/publication/146903 -regional-intratidal-gas-distribution-in-a cute-lung-injury-and-acute-respiratory-dis tress-syndrome-as [gefunden am 2014-04-24]**

**Beschreibung**

Die vorliegende Erfindung betrifft ein Elektroimpedanztomographie-Gerät mit: einer Mehrzahl von um den Thorax eines Patienten anbringbaren Elektroden, einer Steuer- und Auswerteeinheit, die durch Programmierung dazu eingerichtet ist, mindestens ein Elektrodenpaar als einspeisendes Elektrodenpaar mit einem Wechselstrom oder mit einer Wechselspannung zu versorgen, mit mehreren der übrigen Elektrodenpaare jeweils ein Spannungssignal oder Stromsignal als Messsignal aufzunehmen, sukzessive andere Elektrodenpaare als einspeisende Elektrodenpaare fungieren zu lassen, um aus den Messsignalen mit einem Rekonstruktionsalgorithmus eine Matrix aus Bildelementen zu rekonstruieren, die die Verteilung der Impedanzänderungen in der Elektrodenebene repräsentiert, und über einen Atemzug wiederholt Messsignale aufzunehmen und Matrizen zu rekonstruieren, wobei die Steuer- und Auswerteeinheit weiter dazu eingerichtet ist, eine Zeitreihe des globalen Ventilationsverlaufs aus der Folge der rekonstruierten Matrizen als Zeitreihe der mittleren Impedanzänderung oder als eine Zeitreihe eines gemessenen Beatmungsvolumens zu bestimmen und darin eine Inspirations- oder Exspirationsphase zu bestimmen, die Inspirations- oder Exspirationsphase in dem globalen Ventilationsverlauf in eine Anzahl $(1,2,...M_{steps})$ von Schritten gleicher Volumenänderung zu unterteilen, die diesen Schritten gleicher Volumenänderung entsprechenden Zeitpunkte in der Inspirations- oder Exspirationsphase zu bestimmen, für jedes Bildelement die Änderung der lokalen Impedanz zwischen diesen Zeitpunkten zu bestimmen und jeweils das Verhältnis von dieser lokalen Änderung der Impedanz des Bildelements zur globalen gleichen Volumenänderung zu bestimmen, um so eine lokale Folge von relativen Impedanzänderungen des Bildelements als Funktion der Schritte gleicher Volumenänderung zu bilden.

Ein Elektroimpedanztomographie-Gerät (EIT-Gerät) ist zum Beispiel aus EP 1 000 580 A1 bekannt, das zur Aufnahme eines "Elektroimpedanztomographiebildes" eines Thoraxquerschnitts eines Patienten dient.

Die elektrische Impedanztomographie ist ein Verfahren zur Rekonstruktion von Impedanzverteilungen, genauer gesagt von Impedanzänderungen bezüglich einer Referenzverteilung, in elektrisch leitfähigen Körpern. Hierzu wird an der Oberfläche des zu untersuchenden Körpers eine Vielzahl von Elektroden angebracht. In typischen Fällen wird eine ringförmige äquidistante Anordnung von 16 Elektroden verwendet, die mit einem Gurt um den Thorax eines Patienten gelegt werden können. Die Steuer- und Auswerteeinheit verfügt auch über analoge elektrische Schaltungen für die Signalverstärkung und für die Wechselstromeinspeisung und über elektronische Schaltungen zur Digitalisierung und Vorverarbeitung der Spannungssignale und über einen digitalen Signalprozessor zum Steuern des Gerätes und zur Verarbeitung der aufgenommenen Daten zur Rekonstruktion der Impedanzverteilung. Die Steuer- und Auswerteeinheit sorgt dafür, dass aufeinanderfolgend jeweils ein Paar (vorzugsweise) benachbarter Elektroden mit einem elektrischen Wechselstrom versorgt wird (z.B. 5 mA bei 50 kHz) und die elektrischen Spannungen an mehreren verbleibenden Elektrodenpaaren von der Steuer- und Auswerteeinheit erfasst werden (Grundsätzlich kann auch umgekehrt eine Wechselspannung an ein Elektrodenpaar eingespeist und die Wechselströme über mehrere verbleibende Elektrodenpaare gemessen werden); typischerweise werden die Spannungen aller verbleibenden Paare von benachbarten Elektroden erfasst, grundsätzlich ist es aber auch möglich, einzelne Elektroden auszulassen, wodurch allerdings Informationen verloren gehen. Aus der Gesamtheit aller Messsignale bei den aufeinanderfolgenden Stromeinspeisungen, bei denen die Position des einspeisenden Elektrodenpaars Schritt für Schritt um den Elektrodenring wandert, kann mit Algorithmen die Impedanzverteilung, genauer gesagt deren Änderung gegenüber einer Referenzverteilung (z.B. die Impedanzverteilung bei der ersten Aufnahme), rekonstruiert werden. Die bekannten Algorithmen liefern als Rekonstruktionsergebnis eine Matrix aus 32x32 Bildelementen, wobei die Matrix für jedes Bildelement die rekonstruierte Impedanzänderung für dieses Bildelement enthält. Es wird während jedes Atemzugs eine Vielzahl von solchen Matrizen in vorgegebenen zeitlichen Abständen aufgenommen. Diese werden aufeinanderfolgend auf einer Anzeige zur Anzeige gebracht, wodurch der intratidale zeitliche Verlauf der Impedanzverteilung praktisch als Film sichtbar gemacht wird.

Die Thorax-Elektroimpedanztomographie zur Messung der regionalen Lungenventilation findet zunehmende Verbreitung in der forschungsinteressierten Intensivmedizin. Theoretische Modelle und experimentelle Vergleiche von EIT- mit CT-Aufnahmen des Thorax zeigen eine nahezu vollständige Proportionalität von Luftgehalt des Lungengewebes und dessen Impedanz. Die Atemzüge werden räumlich mit etwa 20% des Thoraxdurchmessers und zeitlich typischerweise mit etwa 20 bis etwa 40 Matrizen pro Sekunde aufgelöst, was ein bettseitiges Monitoring der regionalen Lungenventilation ermöglicht. Die Matrizen werden gelegentlich auch als Bilder der Impedanzverteilung (mit 32x32 = 1024 Bildelementen) oder als Frames bezeichnet.

Es wird also für jedes Bildelement über eine Inspirations- oder Exspirationsphase eine Folge von Impedanzänderungen bestimmt, die hier auch als Zeitreihe von Impedanzänderungswerten für dieses Bildelement bezeichnet wird. Im Folgenden werden die Begriffe Zeitreihen von Impedanzänderungswerten und Impedanzänderungskurven mit gleicher Bedeutung verwendet, obwohl eine Zeitreihe aus diskreten Punkten im strengen Sinne keine Kurve ist. Auch in den Darstellungen werden die Zeitreihen aus Darstellungsgründen in Kurvenform als Funktionen der Zeit dargestellt.

Ein wesentlicher Vorteil der hohen Frame-Rate besteht darin, dass die Atemzüge, insbesondere deren Inspirations- und Exspirationsphase, zeitlich aufgelöst werden können. Es ist daher nicht nur möglich, die regionale Verteilung der ventilierten Luft am end-inspiratorischen Zustand (Tidal-Bild) zu analysieren, sondern auch das zeitliche Verhalten während der Inspiration und Exspiration zu untersuchen, um daraus Rückschlüsse über regionale lungenmechanische Abläufe zu gewinnen. So werden zum Beispiel in dem Artikel "Regional Ventilation delay index: detection of tidal recruitment using electric impedance tomography", T. Muders et al, Vincent JL, editor, Yearbook of Intensive Care and Emergency Medicine, lokale Inspirationskurven bestimmt und für jede lokale Inspirationskurve jeweils die Zeit, zu der sie 40% ihres Maximalwertes erreicht hat, ins Verhältnis zur globalen Inspirationszeit gesetzt und daraus ein Bild von schnelleren oder langsameren Regionen mit kleineren oder größeren Zeitkonstanten als im Durchschnitt generiert. Daraus wird ein "Regional ventilation delay-index (RVD) als Maß für die zeitliche Inhomogenität definiert.

In EP 2 228 009 A1 wird einen Schritt weitergegangen und die globale Inspirations- oder Exspirationsphase in mehrere äquidistante Volumenschritte unterteilt und die intratidalen Variationen (ITV), also die Umverteilung der ventilierten Teilvolumina pro Volumenschritt, in sogenannten "regions of interest" (ROI) bestimmt, die eine definierte Anzahl von Bildelementen umfassen. Im Zusammenhang mit den ROI werden auch einzelne Bildelemente erwähnt. Das Ergebnis wird dann in für die ROIs spezifischen Kurven über die äquidistanten Volumenschritte oder daran gekoppelte Größen dargestellt. So wird ermittelt, welche Lungengebiete zu welchem Zeitpunkt der In- oder Exspiration mehr oder weniger Luft erhalten als die anderen Gebiete. Im idealen zeitlich homogenen Fall bleibt alles konstant. Bei zeitlich inhomogenen Lungen kommt es dagegen zu Umverteilungen. Der Vorteil der höheren Unterteilung der Respirationsflanken des ITV im Vergleich zum Betrachten des einen 40% Schwellenwertes beim RVD wird dadurch bezahlt, dass man Kurven über räumlich gröbere ROIs betrachtet und kein visuell einfach zugängliches Bild wie beim RVD zur Verfügung hat, sondern eine Vielzahl von Kurven. Zudem können durch ungünstige Wahl der ROIs unter Umständen zeitliche Inhomogenitäten übersehen werden. Des Weiteren wird kein globaler ITV-Parameter analog dem RVD-Index definiert, der auf einen Blick verrät, ob Umverteilungen vorliegen.

Es ist Aufgabe der vorliegenden Erfindung, ein Elektroimpedanztomographie-Gerät und ein entsprechendes Verfahren anzugeben, mit denen intratidale Umverteilungen lokal erfasst und ein als Bild kodiertes Umverteilungsmaß zur schnellen visuellen Erfassbarkeit der lokalen intratidalen Umverteilungen angezeigt werden können.

Zur Lösung dieser Aufgabe dient das Elektroimpedanztomographie-Gerät mit den Merkmalen von Patentanspruch 1 sowie das Verfahren mit den Merkmalen des Patentanspruchs 9. Vorteilhafte Ausführungsformen der Erfindung sind in den Unteransprüchen aufgeführt.

Erfindungsgemäß ist die Steuer- und Auswerteeinheit des EIT-Geräts dazu eingerichtet, in einer Inspirations- oder Exspirationsphase für jedes Bildelement an die lokale Folge der relativen Impedanzänderungen als Funktion der Schrittnummer $(1,2,...M_{steps})$ der gleichen Volumenänderungen eine Gerade anzupassen und deren Nullstelle zu bestimmen, dieser Nullstelle eine Zeit innerhalb der Inspirations- oder Exspirationsphase zuzuordnen und eine Ventilationsgröße zu dieser Zeit als skalares Maß für den Verlauf der lokalen Impedanzänderung dem Bildelement zuzuordnen und das Bildelement in Abhängigkeit von der so bestimmten Ventilationsgröße in Grauwert- oder Farbkodierung auf der Anzeigeeinrichtung zur Anzeige zu bringen.

Die Inspirations- oder Exspirationsphase in dem globalen Ventilationsverlauf wird in eine Anzahl von Schritten $(1,2,...M_{steps})$ gleicher Volumenänderung $\Delta V$ unterteilt, die diesen Schritten gleicher Volumenänderung entsprechenden Zeitpunkte in der Inspirations- oder Exspirationsphase werden bestimmt und dann für jedes Bildelement zu den bestimmten Zeitpunkten jeweils das Verhältnis aus lokaler Impedanzänderung des Bildelements zum globalen Ventilationsverlauf bestimmt. Diese Folge von Verhältniswerten wird dann als Funktion der Volumenänderungsschritte $(1,2,...M_{steps})$ aufgefasst und ausgewertet. Praktisch wird diese Volumenänderung $\Delta V$ pro Schritt bestimmt, indem die gesamte Volumenänderung über die Inspirations- oder Exspirationsphase durch die vorgegebene Anzahl von Schritten $M_{steps}$ geteilt wird. In den Ausführungsbeispielen werden z.B. 8 Schritte verwendet. Das Auswerten der lokalen Zeitreihen bei gleichen Volumenänderungsschritten $\Delta V$ des globalen Ventilationsverlaufs bedeutet praktisch, dass man den lokalen Verlauf der Ventilationsänderung als Funktion des globalen Ventilationsverlaufs aufträgt, d.h. die x-Achse mit den Schritten $(1,2,...M_{steps})$ entspricht den äquidistanten $\Delta V$-Schritten des ansteigenden globalen Ventilationsvolumens bis zum gesamten Inspirationsvolumen und die y-Achse dem Verhältnis aus lokalem variablen Ventilationsänderungsverlauf zur globalen Ventilationsänderung $\Delta V$.

Im weiteren werden folgende Begriffe als Abkürzung verwendet:

- Die relative Änderung zwischen den Schritten m und m+1 der Zeitreihe der lokalen Impedanzänderung eines Bildelementes zum globalen Volumen bzw. Impedanzänderungsschritt $\Delta V$ bzw. $\Delta Z_{glo}$ für einen der m=1, 2..., $M_{steps}$ Schritte wird als (m-ter) lokaler ITV-Wert des Bildelementes bezeichnet (ITV: intratidale-Verteilung).
- Die von m=1 bis $M_{steps}$ geordneten Wertepaare (m=1, erster ITV-Wert) ... (m= $M_{steps}$, $M_{steps}$-ter ITV-Wert) aller lokaler ITV-Werte wird als lokale ITV-Kurve des Bildelementes bezeichnet. Die ITV-Kurven sind somit eine Funktion der ITV-Werte in Abhängigkeit der m=1...$M_{steps}$ Schritte.
- Das skalare Maß der lokalen ITV-Kurve eines Bildelementes wird auch als lokaler ITU-Wert des Bildelementes bezeichnet(ITU: intratidale Umverteilung).

Erfindungsgemäß ist die Steuer- und Auswerteeinheit dazu eingerichtet, in einer Inspirations- oder Exspirationsphase für jedes Bildelement an die ITV-Kurven eine Gerade anzupassen und neben deren Anstieg auch deren Nullstelle zu bestimmen, dieser Nullstelle einen Zeitpunkt innerhalb der Inspirations- oder Exspirationsphase zuzuordnen, diesem Zeitpunkt eine Ventilationsgröße zu diesem Zeitpunkt zuzuordnen und das Bildelement in Abhängigkeit von der so bestimmten Ventilationsgröße durch Grauwert- oder Farbkodierung auf der Anzeigeeinrichtung zur Anzeige zu bringen. Die hinter dieser Vorgehensweise stehende Überlegung besteht darin, dass bei Auftragen des lokalen Impedanzänderungsverlaufs als Funktion der Schrittnummer gleicher globaler Impedanz- oder Volumenänderungsschritte gewissermaßen eine Auftragung als Funktion des globalen Impedanzverlaufs von 0, $\Delta Z_{glo}$, $2 \cdot \Delta Z_{glo}$, $...M_{steps}\cdot \Delta Z_{glo}$ oder des globalen Volumenverlaufs erfolgt. Idealerweise verhält sich der lokale Ventilationsverlauf genauso wie der globale und es ergibt sich eine Gerade parallel zur x-Achse, was bedeutet, daß es keine Unterschiede im zeitlichen Verhalten der lokalen zur globalen Ventilation gibt: kurz zeitlich homogen. Praktisch bedeutet diese Auftragung als Funktion des globalen Ventilationsverlaufs eine Differenzierung der lokalen Kurve nach dem ventilierten globalen Volumen. Durch Aufsuchen der Nullstelle wird damit praktisch ein Extremwert im lokalen Impedanzverlauf als Funktion des Volumens erfasst. Da bei typischer Ventilation das ventilierte Volumen in der Inspirationsphase monoton mit der Zeit steigt bzw. in der Exspirationsphase monoton fällt, entspricht dieses Extremum im Volumen ebenfalls einem in der Zeit. Bei einem positiven Anstieg der angepaßten Geraden in der Inspirationsphase entspricht dieser Extremwert einem Minimum und daher dem Zeitpunkt, an dem sich die Alveolen zu öffnen beginnen, in der Exspirationsphase dem Zeitpunkt, zu dem sich die Alveolen schließen. Vorzugsweise verbindet die Steuer- und Auswerteeinheit mit diesem Zeitpunkt eine Ventilationsgröße, z.B. die globale Zeitreihe der Impedanzänderung oder eine anderweitig bestimmte Zeitreihe des Beatmungsdrucks, des Volumens, des Flows oder des aus ihnen mit einem Lungenmechanik-Modell ermittelten Alveolardrucks. Wenn ein Bild angezeigt wird, in dem jedes Bildelement in Abhängigkeit von der Ventilationsgröße, zu der sich die Alveolen öffnen oder schließen, in entsprechender Kodierung (Farbkodierung oder Grauwertkodierung) angezeigt wird, kann dies dem Arzt oder Personal wichtige Hinweise darauf geben, wie das Beatmungsgerät einzustellen ist, zum Beispiel welcher PEEP-Wert einzustellen ist.

In einer bevorzugten Ausführungsform ist die Steuer- und Auswerteeinheit weiter dazu eingerichtet, für die bestimmten Zeitpunkte gleicher Volumenänderungsschritte jedes Bildelement in Abhängigkeit von der lokalen Impedanzänderung des Bildelements durch Grauwert- oder Farbkodierung auf der Anzeigeeinrichtung zur Anzeige zu bringen. Hier wird also die kumulative Verteilung der Ventilation in mehreren Schritten nach jeweils gleicher Volumenänderung über die Inspirations- oder Exspirationsphase angezeigt. Wenn zum Beispiel acht Schritte gleicher Volumenänderung über die Inspirations- oder Exspirationsphase gewählt, so werden acht Bilder mit den lokalen Impedanzänderungen vom Beginn der Inspiration bis zu deren Ende oder vom Beginn der Exspiration bis zu deren Ende als Folge von EIT-Bildern erzeugt.

In einer alternativen Ausführungsform ist die Steuer- und Auswerteeinheit dazu eingerichtet, ab dem zweiten aus der Anzahl der bestimmten Zeitpunkte für die bestimmten Zeitpunkte gleicher Volumenänderungsschritte jedes Bildelement in Abhängigkeit von der Differenz der lokalen Impedanzänderung und Impedanzänderung zum vorhergehenden Zeitpunkt durch Grauwert- oder Farbkodierung auf der Anzeigeeinrichtung zur Anzeige zu bringen. Dies ergibt dann eine Folge von differentiellen EIT-Bildern, intratidale Verteilungs-Bilder genannt, d.h. für jedes Bildelement jeweils die Veränderung der kumulativen Ventilationsverteilung zum vorhergehenden Schritt.

In einer bevorzugten Ausführungsform ist die Steuer- und Auswerteeinheit weiter dazu eingerichtet, die Rekonstruktion der Matrizen, die Bestimmung der Zeitreihen, der ITV-Kurven und der ITU-Werte (skalaren Maße) für jedes Bildelement in Echtzeit durchzuführen. Alternativ ist die Steuer- und Auswerteeinheit dazu eingerichtet, die Messsignale zu speichern und die Rekonstruktion der Matrizen, die Bestimmung der Zeitreihen für den globalen Ventilationsverlauf und die Bestimmung der lokalen Zeitreihen, der ITV-Kurven und der ITU-Werte für jedes Bildelement zeitverzögert durchzuführen oder die Rekonstruktion der Matrizen in Echtzeit durchzuführen und diese zu speichern und die Bestimmung der Zeitreihen, der ITV-Kurven und ITU-Werte zeitverzögert durchzuführen.

In einer bevorzugten Ausführungsform ist die Steuer- und Auswerteeinheit weiter dazu eingerichtet, die ITU-Werte in einen Farbcode umzusetzen und die Bildelemente farbcodiert auf der Anzeigeeinrichtung zur Anzeige zu bringen.

In einer bevorzugten Ausführungsform ist die Steuer- und Auswerteeinheit dazu eingerichtet, die ITU-Werte in den Bildelementen zu einem globalen Parameter zusammenzufassen und alphanumerisch und/oder graphisch auf der Anzeigeeinrichtung darzustellen. Falls in einem nicht erfindungsgemäßen Fall als ITU-Wert eines Bildelementes die gegebenenfalls mit der Anzahl der Schritte $M_{steps}$ multiplizierte mittlere Steigung verwendet wird, kann als globaler Parameter die Summe über alle positiven oder negativen gegebenenfalls mit der Anzahl der Schritte $M_{steps}$ multiplizierten Anstiege oder die halbierte Summe über die Beträge der gegebenenfalls mit der Anzahl der Schritte $M_{steps}$ multiplizierten Steigungen verwendet werden. In dem ebenfalls nicht erfindungsgemäßen Fall, in dem die Standardabweichung als ITU-Wert eines Bildelementes verwendet wird, kann als globaler Parameter die über alle Bildelemente aufsummierte Standardabweichung oder die gemittelte Standardabweichung verwendet werden. Bei nicht erfindungsgemäßer Verwendung der Maximalwerte der Beträge der gegebenenfalls mit der Anzahl der Schritte $M_{steps}$ multiplizierten lokalen Steigungen als lokaler ITU-Wert kann die Summe über alle Maximalwerte der Beträge der gegebenenfalls mit der Anzahl

der Schritte $M_{steps}$ multiplizierten lokalen Steigungen verwendet werden. Falls die maximale Steigungsänderung innerhalb der Inspirations- oder Exspirationsphase als ITU-Wert verwendet wird (ebenfalls nicht erfindungsgemäß), kann die Summe über alle maximalen gegebenenfalls mit der Anzahl der Schritte $M_{steps}$ multiplizierten Steigungsänderungen als globaler Parameter verwendet werden.

Die Erfindung wird im Folgenden anhand von Ausführungsbeispielen in den Zeichnungen beschrieben, in denen:

Fig. 1 bis 3 schematische Darstellungen von Komponenten oder Modulen des Elektroimpedanztomographie-Gerätes zur Erläuterung von deren Funktionen zeigen, und

Fig. 4 die lokale Impedanzänderung relativ zum Volumenschritt eines Bildelements als Funktion der Schrittnummer einer vorgegebenen Anzahl von Schritten gleicher Volumenschritte zeigt.

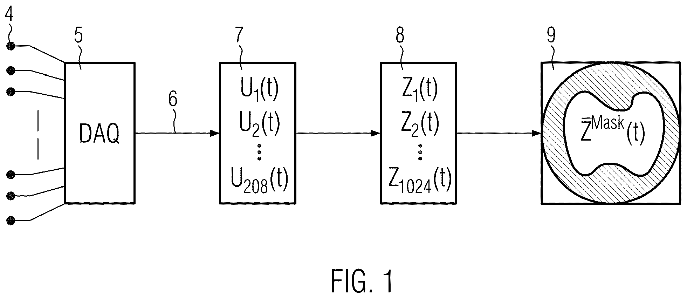

In dem in Fig. 1 gezeigten Ausschnitt aus dem EIT-Gerät wird in diesem Beispiel ein 16-Elektroden-System verwendet, wobei die Elektroden 4 ringförmig um den Thorax angebracht sind. Über die Datenakquisitionseinheit 5 des EIT-Geräts erfolgen Stromeinspeisung über ein Paar (vorzugsweise) benachbarter Elektroden, Spannungsmessungen über mehrere der übrigen benachbarten Elektrodenpaare, dann Stromeinspeisung über ein anderes Paar benachbarter Elektroden, etc. bis mehrere oder alle benachbarten Elektrodenpaare einmal einspeisendes Elektrodenpaar waren; die dabei erhaltenen Messspannungen werden auch als Frame bezeichnet. Ein Frame von Messspannungen besteht hier aus 208 Spannungen, die mit Frame-Raten zwischen 10 Hz Und 50 Hz aufgenommen werden können. Die Anzahl von 208 Messspannungen ergibt sich bei einem 16 Elektrodensystem dann, wenn jedes der 16 Paare benachbarter Elektroden einmal als einspeisendes Elektrodenpaar verwendet wird, wobei es jeweils unter den verbleibenden 14 Elektroden 13 unterschiedliche Paare benachbarter Elektroden gibt, zwischen denen eine Messspannung erfasst wird, so dass insgesamt bei einem Durchlauf 16x13 = 208 Messspannungen erfasst werden. Dies ist eine typische Ansteuerungsweise für ein EIT-Gerät mit einem 16 Elektroden-system. Es ist aber in technischen Umsetzungen von EIT-Geräten auch vorstellbar, dass nicht alle der vorhandenen Elektroden zur Einspeisung von Strom oder Spannung genutzt werden, sondern einzelne Elektroden oder Elektrodenpaare bei der Einspeisung übersprungen werden. Ebenso ist es vorstellbar, dass Spannungsmessungen oder Strommessungen nicht an allen der vorhandenen Elektroden vorgenommen werden, sondern einzelne Elektroden oder Elektrodenpaare übersprungen und bei den Messungen ausgelassen werden.

Im vorliegenden Ausführungsbeispiel werden die 208 Zeitreihen der Spannungen über ein Bussystem 6 zur Speichereinheit 7 des EIT-Geräts oder einer externen Speichereinheit transportiert. Aus den gemessenen Spannungen werden 1024 (relative) Impedanzänderungen $Z_1$, $Z_2$, ...$Z_{1024}$ rekonstruiert, die auf ein Bild mit 32x32 Bildelementen abgebildet werden. Die Rekonstruktion und weitere Analysen können sowohl online als auch zeitlich und räumlich versetzt offline erfolgen. Die im Folgenden entstehenden Daten können daher intern im EIT-System über ein Bussystem oder in einen externen Speicher abgelegt werden. Es wird im Folgenden nicht mehr zwischen beiden Möglichkeiten unterschieden.

Ein Rechenmodul rekonstruiert aus den 208 Zeitreihen der Spannungen $U_1$ (t), $U_2$ (t) , ... $U_{208}$ (t) die 1024 Zeitreihen der relativen Impedanzänderungen ($Z_1(t)$, $Z_2$ (t), ... $Z_{1024}(t)$) und stellt sie in einem lokalen Impedanzmodul 8 bereit. Mit einer vorgegebenen oder aus den Daten bestimmten Bildelement-Maske 9 werden nur die informationstragenden "ventilierten" Bildelemente (Pixel) i = [1,2,...$N_{pix}$] ausgewählt, d.h. die in Fig. 1 angedeuteten Randbereiche 9 außerhalb der Lunge werden ausgeblendet.

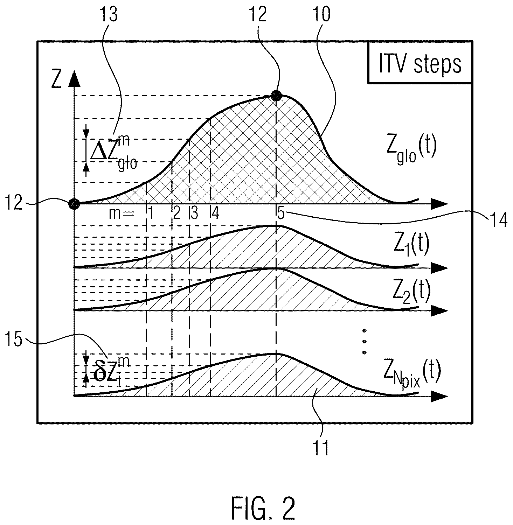

In Fig. 2 ist schematisch ein Analysemodul der Steuer- und Auswerteeinheit gezeigt. In Fig. 2 ist oben schematisch die globale (relative) Impedanzänderungszeitreihen $Z_{glo}(t)$ 10, kurz globale Impedanzkurve, gezeigt, die durch Aufsummierung der lokalen (relativen) Impedanzänderungszeitreihen $Z_i$ (t) bestimmt und gegebenenfalls geeignet normiert wird. Diese globale Impedanzkurve $Z_{glo}$ (t) repräsentiert die globale Impedanzänderung im Unterschied zu der lokalen Impedanzänderung $Z_i$ (t) der einzelnen Bildelemente. Die globale Impedanzänderungszeitreihe oder Impedanzänderungskurve ist aufgrund der nahezu vollständigen Proportionalität zwischen Atemluftvolumenänderung und Impedanzänderung hochgradig mit der Ventilationsvolumenkurve korreliert; im Fall eines den Patienten beatmenden Beatmungsgerätes kann die Impedanzänderungskurve auch auf die Volumenkurve des Beatmungsgerätes normiert werden.

Aufgrund der hohen Korrelation der globalen Impedanzkurve, die in Fig. 2 oben dargestellt ist, mit der Volumenkurve der Atmung kann die globale Impedanzkurve $Z_{glo}$ (t) zur Bestimmung der Inspirations- und Exspirationsphase verwendet werden, d.h. Beginn und Ende der Inspirationsflanke und Beginn und Ende der Exspirationsflanke können durch die Steuer- und Auswerteeinheit in der globalen Impedanzkurve bestimmt werden. Fig. 2 zeigt schematisch das Analysemodul der Steuer- und Auswerteeinheit, in dem die globale Impedanzkurve $Z_{glo}$ (t) und die lokalen Impedanzkurven $Z_1$ (t), $Z_2$ (t), ... , $Z_{Npix}$ (t) aufgenommen und analysiert werden. In der globalen Impedanzkurve $Z_{glo}$ (t) ist die Inspirationsphase bestimmt und durch die Anfangs- und Endpunkte 12 markiert.

Die folgende Beschreibung in Bezug auf Fig. 2 betrifft Merkmale, die nicht unter die Patentansprüche fallen.

In dem Beispiel wird für die Inspirationsphase die mit der Anzahl der ITV-Schritte $M_{steps}$ multiplizierte mittlere Steigung als skalares Maß (ITU-Wert) der ITV-Kurven gewählt. Dieser Parameter gibt trotz der Informationsreduktion der Kurvenpunkte auf nur einen Wert eine grundlegende Information wieder: welche Lungengebiete am Anfang der Inspiration weniger und am Ende mehr der eingeatmeten Luft erhalten oder umgekehrt und wie groß die Umverteilung der respirierten Luftteilvolumina bei einem Atemzug ist. Die Darstellung der Bildelemente in Abhängigkeit von diesem Parameter wird im folgenden als ITU-Map (intratidale Umverteilungs-Map) bezeichnet. Als globale Größe wird die halbe Summe der Beträge der Werte der ITU-Map gewählt, da dieser eine einfache Interpretation als relativer Anteil der umverteilten Menge der respirierten Luftteilvolumina darstellt.

Die Inspirationsflanke zwischen Anfangs- und Endzeitpunkt der Inspiration der globalen Impedanzkurve wird gemäß dem in EP 2 228 009 A1 beschriebenen ITV-Verfahren in $M_{steps}$ äquidistante globale Impedanzänderungsschritte $\Delta Z^m_{glo}$=const., m=1...$M_{steps}$, unterteilt, angedeutet in Fig. 2 durch die waagerechten Punktlinien bei 13, die alle gleichen Abstand haben, d.h. gleichen Volumenänderungsschritten oder Luftteilvolumina der Inspiration entsprechen. In Fig. 2 ist die Anzahl der Schritte mit $M_{steps}$ = 5 gewählt. Die Schrittgröße $\Delta Z^m_{glo}$ kann als eingeatmetes Luftteilvolumen betrachtet werden, wobei aufgrund der hohen Korrelation der Impedanzänderungskurve mit der Volumenkurve der Atmung die Unterteilung in $M_{steps}$ Impedanzänderungsschritte einer Unterteilung der Volumenkurve der Inspirationsphase in $M_{steps}$ gleiche Volumenänderungsschritte $\Delta V$ entspricht. Im Zeitbereich liegen die entsprechenden Schritte i.A. nicht äquidistant auseinander, angedeutet durch die senkrechten Strichlinien bei den Schritten 1, 2, 3, 4 und 5 aufgrund i.A. nicht konstanten Flows dV/dt. Die Zeitpunkte, die den gleichen Volumenschritten entsprechen und in Figur 2 durch die vertikalen gestrichelten Linien angedeutet sind, werden als die Zeitpunkte oder Stützstellen verwendet, um die lokale Impedanzänderungskurve auszuwerten und deren Zeitreihen aufzustellen. Das Auswerten der lokalen Impedanzänderungskurven nach gleich Volumenänderungsschritte $\Delta V$ kann praktisch so interpretiert werden, das man den lokalen Ventilationsverlauf als Funktion des globalen Ventilationsverlaufs von 0, $\Delta V$, $2 \cdot \Delta V$, ... $M_{steps} \cdot \Delta V$ aufträgt.

Für jedes Bildelement i und jeden Schritt m wird die lokale Impedanzänderung $\delta Z_i^m$ herangezogen, die mit der globalen Impedanzänderung $\Delta Z^m_{glo}$ zu diesem Schritt zum lokalen ITV-Wert $\zeta_i^m$ normiert wird:

$$\zeta_i^m = \frac{\delta Z_i^m}{\Delta Z_{glo}^m} \cdot 100,$$

wobei wegen $\sum_{i=1}^{Npix} \delta Z_i^m = \Delta Z_{glo}^m$ für alle Schritte m = 1, ... $M_{steps}$ $\sum_{i=1}^{Npix} \zeta_i^m = 100\%$ gilt.

Für jedes Bildelement i wird für die m = 1,... $M_{steps}$ ITV-Werte $\zeta_i^m$ die Steigung $S_i$ einer an die $\zeta_i^m$ Werte angepassten Gerade durch quadratische Fehlerminimierung bestimmt und mit der Anzahl der ITV-Schritte $M_{steps}$ multipliziert:

$$S_i = \frac{\sum_{m=1}^{M_{steps}} \zeta_i^m \sum_{m=1}^{M_{steps}} m - M_{steps} \sum_{m=1}^{M_{steps}} \zeta_i^m m}{\left(\sum_{m=1}^{M_{steps}} m\right)^2 - M_{steps} \sum_{m=1}^{M_{steps}} m^2} \cdot M_{steps}$$

Die Multiplikation mit der Anzahl der ITV-Schritte $M_{steps}$ bewirkt zum einen Unabhängigkeit der Werte $S_i$ von der Anzahl der ITV-Schritte bis auf statistische Schwankungen und zum anderen eine einfache Interpretierbarkeit der Größe als mittlere lokale intratidale Umverteilung der eingeatmeten Inspirationsteilvolumina von Anfang bis Ende der Inspiration (oder der ausgeatmeten Luftteilvolumina über die Exspiration). Daher wird dieser Wert $S_i$ als lokaler oder regionaler ITU-Wert (intratidaler Umverteilungswert) bezeichnet.

So bedeutet beispielsweise ein lokaler ITU-Wert von $S_i$=1.5 %, dass während der Inspiration im Mittel von Anfang bis Ende der Anteil der eingeatmeten Luft für diesen Pixel um 1.5% anstieg. Das bedeutet, dass am Anfang also weniger Luft in die Region des Bildelements ging als am Ende, was auf Veränderung der lokalen Zeitkonstanten des Kompartimentes während des Atemzuges, im Extremfall sogar auf tidales Recruitment hindeuten kann.

Lokale ITU-Werte (für ein Bildelement) über 1% sind bereits als recht groß einzustufen. Für ITU-Werte größerer Regionen (ROI's) werden die lokalen ITU-Werte, die in dieser ROI liegen, aufsummiert. Eine ROI (Region of Interest), die z.B. 50 Bildelemente einer z.B. 300 Bildelemente umfassenden Bildelementmaske eines EIT-Bildes mit 32x32 Bildelementen enthält, hätte dann einen regionalen ITU-Wert von mehr als 50%, wenn die einzelnen Bildelemente lokale ITU-Werte von 1% hättten. Das bedeutet, dass in dieser ROI alleine 50% der eingeatmeten Luftteilvolumina umverteilt werden, also dass diese Region z.B. am Ende 50% mehr (oder weniger) Anteil am eingeatmeten Luftteillvolumen als am Anfang erhält, was auf tidales Recruitment oder tidale Überdehnung oder in dieser Lungenregion auf sich stark voneinander unterscheidende Zeitkonstanten in verschiedenen Lungengebieten aufgrund z.B. einer COPD-Erkrankung hindeuten würde.

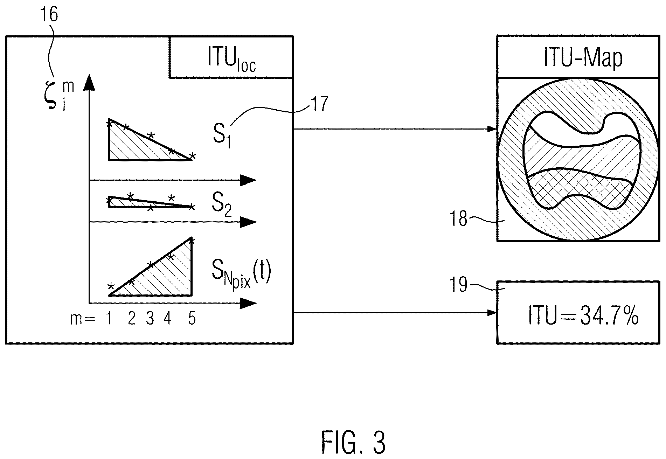

Die ITU-Werte $S_i$, i=1...$N_{pix}$ werden mittels einer Farbkodierung als Bild (ITU-Map) von gleicher Größe als Bild mit 32x32 Bildelementen angezeigt, wie in Fig. 3 schematisch in der Anzeigeeinrichtung 18 angedeutet.

Aufgrund der obiger Definition von $\zeta_i^m$ gilt:

$$\sum_{i=1}^{N_{pix}} \left( \sum_{m=1}^{M_{steps}} \zeta_i^m \sum_{m=1}^{M_{steps}} m - M_{steps} \sum_{m=1}^{M_{steps}} \zeta_i^m m \right) = \sum_{m=1}^{M_{steps}} m \sum_{m=1}^{M_{steps}} \sum_i^{Npix} \zeta_i^m - M_{steps} \sum_{m=1}^{M_{steps}} m \sum_i^{Npix} \zeta_i^m$$

$$= \left( \sum_{m=1}^{M_{steps}} m \right) \cdot M_{steps} \cdot 100 - M_{steps} \left( \sum_{m=1}^{M_{steps}} m \right) \cdot 100 = 0$$

und somit folgt $\sum_{i=1}^{N_{pix}} S_i = 0$ , was auch anschaulich klar ist, denn wenn in ein Lungengebiet ein relativ höherer Anteil des (konstanten) eingeatmeten Luftteilvolumens eingeht, muss in einem anderen Lungengebiet zwangsläufig ein geringerer Anteil des Luftteilvolumens einfließen, wenn die Gesamtsumme eine konstante, nämlich $\Delta V$, ist.

Als globaler ITU-Wert oder kurz ITU wird daher die Hälfte der Betragssumme definiert:

$$\mathrm{ITU} = \frac{\sum_{i=1}^{N_{pix}} |S_i|}{2} \ ,$$

[ITU] =%

Dieser Wert ist relative intratidale Umverteilung der eingeatmeten Luftteilvolumina vom Anfang bis zum Ende der Inspiration (oder Exspiration). Der Wertebereich liegt zwischen 0% ≤ ITU ≤ 100%. Die untere Grenze bezeichnet zeitlich (nicht notwendigerweise räumlich) vollkommen homogene Ventilation, was bedeutet, dass überall in der Lunge gleiche Zeitkonstanten vorliegen. Das heißt zu jeder Phase während der Inspiration (Exspiration) erhalten die verschiedenen ventilierten Lungenregionen stets den gleichen Anteil des ventilierten Luftteilvolumens. Die obere Grenze kennzeichnet maximale Umverteilung. Das heißt zu Anfang der Inspiration geht alles in ein Gebiet der Lunge, das andere erhält nichts, am Ende alles in das andere Gebiet, das erste erhält nichts: Während der Inspiration (Exspiration) werden die ventilierten (differentiellen) Luftteilvolumina also regional vollkommen umverteilt.

Eine Autoskalierung der ITU-Maps ist nicht empfehlenswert, da Bilder mit kleinen lokalen ITU-Werten von etwa $S_i$ <0.1% gleich dargestellt würden wie ITU-Maps mit großen lokalen ITU-Werten von $S_i$ > 1.0%. Es ist daher' ratsam einen gemeinsamen unteren Bereich einer Minimalskalierung zu definieren. Dies erfordert Untersuchung von Patienten und Probanden um den Wertebereich der ITU-Werte näher zu spezifizieren. Aus empirischen Untersuchungen ergab sich das lokale ITU-Werte von $S_i$>0. 75% bereits sehr groß und selten sind, so dass zu empfehlen ist, eine gemeinsame untere ITU-Map-Skala von einem Bereich von etwa - 0.75% < $S_i$ < +0.75% (oder größer) zu verwenden.

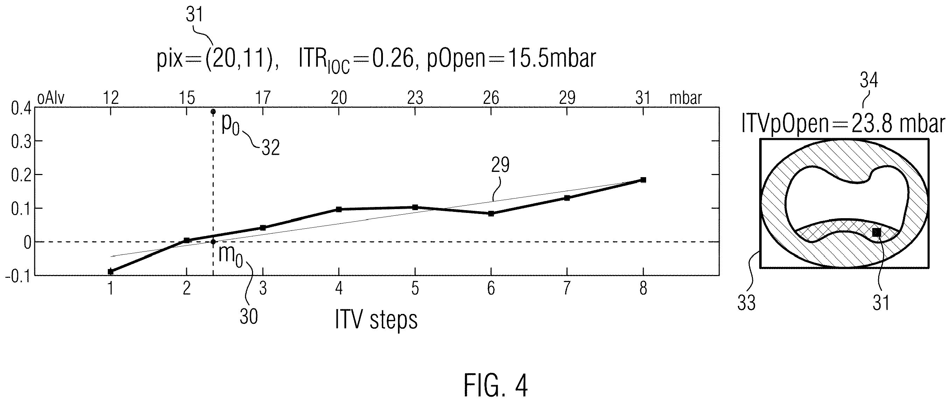

Aus den lokalen $S_i$-Werten, die der ITU-Map zugrunde liegen, kann erfindungsgemäß eine weitere für die Beatmung interessante Größe abgeleitet werden. In Fig. 3 ist beispielhaft der $\zeta_i^m$-Graph des ersten, zweiten und N-ten Bildelements dargestellt. Betrachtet man die unterste der drei $\zeta_i^m$-Graphen in Box 16 in Figur 3, so findet man, dass die an die Daten angepasste Kurve steigend ist. Da in diesem Ausführungsbeispiel ein Geraden-Fit als Anpassung verwendet wird, handelt es sich um eine positive Steigung ($S_i/M_{step}$) . In Fig. 4 ist dies für einen realen Datensatz aus einem Fallbeispiel verdeutlicht. Im Folgenden wird auf die Figur 4 Bezug genommen.

Aus der angepassten Geraden 29 wird erfindungsgemäß die Nullstelle $m_i^0$ 30 des Bildelements i=(20,11) = 20*32+11=651 bestimmt, das mit 31 bezeichnet ist. In diesem Datensatz lagen die Beatmungsdruckkurven vor. Mittels eines Ein-Kompartiment-Modells, welches die Druckabfälle durch Turbulenzen am Mundstück und durch Reibung in den oberen Atemwegen berücksichtigt, wurde aus der Beatmungsdruckskurve die Alveolardruckkurve bestimmt. Der Nullstelle $m_i^0$=2.3 entspricht ein Alveolardruck für den Lungenbereich des Bildelements i=(20,11) von $p_i^0$=15.5 mbar. Dieser Druck kann im Zusammenhang mit dem bekannten Lungenschaden in diesem Fallbeispiel als Öffnungsdruck dieser Lungenregion interpretiert werden und wird im folgenden auch so bezeichnet. Das Verfahren wird für alle Pixel mit positiven ITU-Werten durchgeführt, wobei sinnlose Öffnungsdruckwerte mit $p_i^0 < 0$ auf 0 gesetzt werden. Wenn der Nulldurchgang innerhalb des Tidalhubes wie in diesem Beispiel (min $(p_{alv})$ =12 mbar < $p_i^0$=15.5 mbar < max($p_{alv}$)=31 mbar) erfolgt, ist dies ein starkes Indiz auf tidales Recruitment, also dass sich die Alveolen zyklisch während der Inspiration öffnen und am Ende der Exspiration wieder kollabieren - ein Prozess der aufgrund hoher Spannungskräfte zur Zerstörung der Alveolen führt und vermieden werden muss. Dieser Fall kann auch ohne Vorliegen der Druckinformation bestimmt werden, weil dies nur eine Nullstelle im Bereich $1 < m_i^0 < M_{steps}$ erfordert. Die eventuell vorliegende zugehörige Alveolardruckinformation gibt an, ab welchem PEEP die Alveolen offen bleiben.

Wenn die Nullstelle $m_i^0 < 1$ ist, also vor dem Beginn des Tidalhubs liegt, kann durch Extrapolation des funktionalen Zusammenhangs zwischen den ITV-Schritten m und den zugehörigen Alveolardrücken $p_{alv}(m)$ immer noch ein Wert $p_i^0 > 0$ bei PEEP-Werte>0 existieren (PEEP: "Positive End Exspiratory Pressure"). In diesem Fall liegt dann wahrscheinlich kein tidales Recruitment vor, aber der Öffnungsdruckwert gibt an, ab welchen kleineren PEEP--Werten als verwendeten in etwa die entsprechenden Lungengebiete kollabieren würden.

Die pixelweise bestimmten Öffnungsdrücke pi0, i=1...1024, können als Öffnungsdruckbild 33 kodiert dargestellt werden. Aus dem Öffnungsdruckbild wird aus den $p_i^0$-Werten, die größer sind als 0, ein globaler Öffnungsdruckwert 34 abgeleitet, hier aus dem Mittelwert des oberen Quantils.

### Patentansprüche

1. Elektroimpedanztomographie-Gerät mit: einer Mehrzahl von um den Thorax eines Patienten anbringbaren Elektroden, einer Steuer- und Auswerteeinheit, die durch Programmierung dazu eingerichtet ist, mindestens ein Elektrodenpaar als einspeisendes Elektrodenpaar mit einem Wechselstrom oder mit einer Wechselspannung zu versorgen, mit mehreren der übrigen Elektrodenpaare aus der Mehrzahl der Elektroden jeweils ein Spannungssignal oder Stromsignal als Messsignal aufzunehmen und sukzessive andere Elektrodenpaare aus der Mehrzahl von Elektrodenpaaren als einspeisende Elektrodenpaare fungieren zu lassen, um aus den Messsignalen mit einem Rekonstruktionsalgorithmus eine Matrix aus Bildelementen zu rekonstruieren, die die Verteilung der Impedanzänderungen in der Elektrodenebene repräsentiert, und über die Zeit wiederholt Messsignale aufzunehmen und Matrizen zu rekonstruieren, und mit einer Anzeigeeinrichtung, wobei die Steuer- und Auswerteeinheit weiter dazu eingerichtet ist, eine Zeitreihe des globalen Ventilationsverlaufs aus der Folge der rekonstruierten Matrizen als Zeitreihe der mittleren Impedanzänderung oder als eine Zeitreihe eines gemessenen Beatmungsvolumens zu bestimmen und darin eine Inspirations- oder Exspirationsphase zu bestimmen, die Inspirations- oder Exspirationsphase in dem globalen Ventilationsverlauf in eine Anzahl (1,2,...$M_{steps}$) von Schritten gleicher Volumenänderung zu unterteilen, die diesen Schritten gleicher Volumenänderung entsprechenden Zeitpunkte in der Inspirations- oder Exspirationsphase zu bestimmen, für jedes Bildelement die Änderung der lokalen Impedanz zwischen diesen Zeitpunkten zu bestimmen und jeweils das Verhältnis von dieser lokalen Änderung der Impedanz des Bildelements zur globalen gleichen Volumenänderung zu bestimmen, um so eine lokale Folge von relativen Impedanzänderungen des Bildelements als Funktion der Schritte gleicher Volumenänderung zu bilden, **dadurch gekennzeichnet, dass** die Steuer- und Auswerteeinheit weiter dazu eingerichtet ist, in einer Inspirations- oder Exspirationsphase für jedes Bildelement an die lokale Folge der relativen Impedanzänderungen als Funktion der Schrittnummer (1,2,...$M_{steps}$) der gleichen Volumenänderungen eine Gerade anzupassen und deren Nullstelle zu bestimmen, dieser Nullstelle eine Zeit innerhalb der Inspirations- oder Exspirationsphase zuzuordnen und eine Ventilationsgröße zu dieser Zeit als skalares Maß für den Verlauf der lokalen Impedanzänderung dem Bildelement zuzuordnen und das Bildelement in Abhängigkeit von der so bestimmten Ventilationsgröße in Grauwert- oder Farbkodierung auf der Anzeigeeinrichtung zur Anzeige zu bringen.

2. Elektroimpedanztomographie-Gerät nach Anspruch 1, **dadurch gekennzeichnet, dass** die Steuer- und Auswerteeinheit weiter dazu eingerichtet ist, als Ventilationsgröße die globalen Zeitreihe der Impedanzänderung, Volumen, Flow oder Beatmungsdruck aus einem Beatmungsgerät oder aus diesen Größen über ein Modell berechneten Alveolardruck zu verwenden.

3. Elektroimpedanztomographie-Gerät nach einem der vorhergehenden Ansprüche, **dadurch gekennzeichnet, dass** die Steuer- und Auswerteeinheit weiter dazu eingerichtet ist, für die bestimmten Zeitpunkte gleicher Volumenänderungsschritte jedes Bildelement in Abhängigkeit von der lokalen Impedanzänderung durch Grauwert- oder Farbkodierung auf der Anzeigeeinrichtung zur Anzeige zu bringen.

4. Elektroimpedanztomographie-Gerät nach einem der Ansprüche 1 bis 2, **dadurch gekennzeichnet, dass** die Steuer- und Auswerteeinheit weiter dazu eingerichtet ist, ab dem zweiten aus der Anzahl der bestimmten Zeitpunkte für die bestimmten Zeitpunkte gleicher Volumenänderungsschritte jedes Bildelement in Abhängigkeit von der Differenz der lokalen Impedanzänderung des Bildelements und der lokalen Impedanzänderung zum vorhergehenden Zeitpunkt durch Grauwert- oder Farbkodierung auf der Anzeigeeinrichtung zur Anzeige zu bringen.

5. Elektroimpedanztomographie-Gerät nach einem der vorhergehenden Ansprüche, **dadurch gekennzeichnet, dass** die Steuer- und Auswerteeinheit weiter dazu eingerichtet ist, die Rekonstruktion der Matrizen, die Bestimmung der Zeitreihen und des skalaren Maßes für den Verlauf der lokalen Zeitreihe für jedes Bildelement in Echtzeit durchzuführen.

6. Elektroimpedanztomographie-Gerät nach einem der Ansprüche 1 bis 4, **dadurch gekennzeichnet, dass** die Steuer- und Auswerteeinheit weiter dazu eingerichtet ist, die Messsignale zu speichern und die Rekonstruktion der Matrizen, die Bestimmung der Zeitreihen und des skalaren Maßes für den Verlauf der lokalen Zeitreihe für jedes Bildelement zeitverzögert durchzuführen oder die Rekonstruktion der Matrizen in Echtzeit durchzuführen und diese zu speichern und die Bestimmung der Zeitreihen und des skalaren Maßes für den Verlauf der lokalen Zeitreihe für jedes Bildelement zeitverzögert durchzuführen.

7. Elektroimpedanztomographie-Gerät nach einem der vorhergehenden Ansprüche, **dadurch gekennzeichnet, dass** die Steuer- und Auswerteeinheit weiter dazu eingerichtet ist, die skalaren Maße für den Verlauf der lokalen Zeitreihen in einen Farbcode umzusetzen und die Bildelemente farbcodiert auf der Anzeigeeinrichtung zur Anzeige zu bringen.

8. Elektroimpedanztomographie-Gerät nach einem der vorhergehenden Ansprüche, **dadurch gekennzeichnet, dass** die Steuer- und Auswerteeinheit weiter dazu eingerichtet ist, die skalaren Maße für den Verlauf der lokalen Impedanzänderung in den Bildelementen zu einem globalen Parameter zusammenzufassen und alphanumerisch und/oder graphisch auf der Anzeigeeinrichtung darzustellen,

9. Verfahren zur Aufnahme einer Folge von EIT-Bildern einer Querschnittsebene des Thorax eines Patienten durch eine Vielzahl von auf dem Umfang des Thorax verteilte Elektroden, wobei bei dem Verfahren
mindestens ein Elektrodenpaar als einspeisendes Elektrodenpaar mit einem Wechselstrom oder einer Wechselspannung versorgt wird, mit mehreren der übrigen Elektrodenpaare aus der Vielzahl der Elektrodenpaare jeweils ein Spannungssignal oder Stromsignal als Messsignal aufgenommen wird und sukzessive andere Elektrodenpaare aus der Vielzahl von Elektrodenpaaren als einspeisende Elektrodenpaare betrieben werden,
aus der Gesamtheit der Messsignale mit einem Rekonstruktionsalgorithmus eine Matrix aus Bildelementen rekonstruiert wird, die die Verteilung der Impedanzänderungen in der Elektrodenebene repräsentiert,
über die Zeit wiederholt Matrizen der Impedanzänderung rekonstruiert werden,
eine Zeitreihe des globalen Ventilationsverlaufs aus der Folge der rekonstruierten Matrizen als Zeitreihe der mittleren Impedanzänderung oder als eine Zeitreihe eines gemessenen Beatmungsvolumens bestimmt wird,
in dem globalen Ventilationsverlauf eine Inspirations- oder Exspirationsphase bestimmt, die Inspirations- oder Exspirationsphase in eine Anzahl $(1,2,...M_{steps})$ von Schritten gleicher Volumenänderung unterteilt wird und die diesen Schritten gleicher Volumenänderung entsprechenden Zeitpunkte in der Inspirations- oder Exspirationsphase bestimmt werden,
für jedes Bildelement zu den bestimmten Zeitpunkten gleicher Volumenänderung das Verhältnis aus Impedanzänderung des Bildelements zwischen diesen Zeitpunkten zur gleichen globalen Volumenänderung gebildet wird und diese Verhältnisse als Folge der lokalen relativen Impedanzänderung in Abhängigkeit von den zugehörigen Schrittnummern des Bildelements bestimmt wird,
**dadurch gekennzeichnet, dass**
in einer Inspirations- oder Exspirationsphase für jedes Bildelement an die lokale Folge der relativen Impedanzänderungen als Funktion der Schrittnummer $(1,2,...M_{steps})$ der gleichen Volumenänderungen eine Gerade anzupassen und deren Nullstelle bestimmt wird, dieser Nullstelle eine Zeit innerhalb der Inspirations- oder Exspirationsphase zuzuordnet wird und eine Ventilationsgröße zu dieser Zeit als skalares Maß für den Verlauf der lokalen Impedanzänderung dem Bildelement zugeordnet wird und das Bildelement in Abhängigkeit von der so bestimmten Ventilationsgröße in Grauwert- oder Farbkodierung auf der Anzeigeeinrichtung zur Anzeige gebracht wird.

## Claims

1. An electric impedance thomography device comprising: a plurality of electrodes which can be arranged about the chest of a patient, and a control and analyzing unit which is, by progamming, configured to supply at least one electrode pair as a feeding electrode pair with an alternating current or with an alternating voltage, to record a voltage signal or a current signal as a measured signal with a plurality of the remaining electrode pairs from the plurality of electrodes, and to let other electrode pairs of the plurality of electrode pairs act consecutively as the feeding electrode pair, to reconstruct from the measured signals, with a reconstruction algorithm, a matrix of image elements which represent the distribution of the impedance changes in the electrode plane, and to repeatedly record measured signals over time and to reconstruct matrices, and to display them with a display device, wherein the control and analyzing unit is further configured to determine a time series of a global ventilation curve from the sequence of the reconstructed matrices as a time series of the mean impedance change or as a time series of a measured respiration volume and to determine an inspiration or expiration phase therein, to divide the inspiration or expiration phase of the global ventilation curve into a number (1, 2, ... $M_{steps}$) of steps of equal volume change, to determine times corresponding to these steps of equal volume change in the inspiration or expiration phase, to determine, for each image element, the change in the local impedance between these times and to determine each time the ratio of this local change in impedance of the image element to the global equal volume change to thereby form a local sequence of relative impedance changes of the image element as a function of the number of steps of equal volume change, **characterized in that** the control and analyzing unit is further configured to fit, for each image element in an inspiration or expiration phase a straight line to the local sequence of relative impedance changes as a function of the step number (1, 2, ... $M_{steps}$) of equal volume changes and to determine the zero point of the straight line, to assign to this zero point a time within the inspiration or expiration phase, and to assign to the image element a ventilation variable at this time, as a scalar indicator for the curve of the local impedance change, and to display the image element as a function of the ventilation variable thus determined in gray scale value or color coding on the display device.

2.  An electric impedance tomography device according to claim 1, **characterized in that** the control and analyzing unit is configured to use as the ventilation variable the global time series of the impedance change, volume, flow or respiration pressure from a respirator or the alveolar pressure calculated from these variables by means of a model.

3.  An electric impedance tomography device according to any of the preceding claims, **characterized in that** the control and analyzing unit is further configured to display each image element for the determined times of equal volume change steps as a function of the local impedance change by gray scale value or color coding on the display device.

4.  An electric impedance tomography device according to any of the claims 1 to 2, **characterized in that** the control and analyzing unit is configured to display each image element beginning from the second of the number of times determined for the determined times of equal volume change steps as a function of the difference of the local impedance change of the image element and the local impedance change from the preceding time by gray scale value or color coding on the display device.

5.  An electric impedance tomography device according to any of the preceding claims, **characterized in that** the control and analyzing unit is further configured to perform the reconstruction of the matrices, the determination of the time series and of the scalar indicator of the curve of the local time series for each element in real time.

6.  An electric impedance tomography device according to any of the claims 1 to 4, **characterized in that** the control and analyzing unit is further configured to store the measured signals and to perform the reconstruction of the matrices, the determination of the time series and of the scalar indicator of the curve of the local time series for each image element with a time delay or to perform the reconstruction of the matrices in real time and to store these, and to perform the determination of the time series and of the scalar indicator of the curve of the local time series for each image element with a time delay.

7.  An electric impedance tomography device according to any of the preceding claims, **characterized in that** the control and analyzing unit is further configured to convert the scalar indicator of the curve of the local time series into a color code and to display the image element in a color-coded form on the display device.

8.  An electric impedance tomography device according to any of the preceding claims, **characterized in that** the control and analyzing unit is further configured to combine the scalar indicators for the curve of the local impedance change in the image elements into a global parameter and to display it alphanumerically and/or graphically on the display device.

9.  A method for recording a sequence of EIT images of a cross-sectional plane of the chest of a patient by a plurality of electrodes distributed over the circumference of the chest, wherein the method comprises
    supplying at least one electrode pair as a feeding electrode pair with an alternating current or with an alternating voltage, recording a voltage signal or a current signal as a measured signal with a plurality of the remaining electrode pairs from the plurality of electrode pairs and operating consecutively other electrode pairs of the plurality of electrode pairs as feeding electrode pair,

reconstructing a matrix of image elements from all measured signals with a reconstruction algorithm, which image elements represent the distribution of the impedance changes in the electrode plane,

repeatedly reconstructing the matrices of the impedance change over time,

determining a time series of the global ventilation curve from the sequence of reconstructed matrices, as a time series of the mean impedance change or as time series of a measured respiration volume,

determining in the global ventilation curve an inspiration or an expiration phase, wherein the inspiration or expiration phase is divided into a number of steps of equal volume change, and determining the times corresponding to these steps of equal volume change during the inspiration or expiration phase,

for each image element forming, at the determined times of equal volume change, the ratio of the impedance change of the image element between the times to the equal global volume change, and determining these ratios as a sequence of the local relative impedance change as a function of the corresponding step numbers of the image element,

**characterized in that**

in a inspiration or expiration phase for each image element a straight line is fitted to the local sequence of the relative impedance changes as a function of the step number (1, 2, ... $M_{steps}$) of equal volume changes and its zero point is determine, to this zero point a time within the inspiration or expiration phase is assigned, and a ventilation variable at this time is assigned as a scalar indicator for the local impedance change to the image element, and the image element is displayed as a function of the ventilation variable does determine in gray scale value or color coding on the display device.

## Revendications

1. Appareil de tomographie par impédance électrique avec : une pluralité d'électrodes pouvant être fixées autour du thorax d'un patient, une unité de commande et d'évaluation, qui est conçue par programmation pour alimenter au moins une paire d'électrodes comme paire d'électrodes d'injection avec un courant alternatif ou avec une tension alternative, pour recevoir respectivement un signal de tension ou signal de courant en tant que signal de mesure avec plusieurs des paires d'électrodes restantes parmi la pluralité des électrodes et pour faire fonctionner d'autres paires d'électrodes successives parmi la pluralité de paires d'électrodes comme paires d'électrodes d'injection, afin, à partir des signaux de mesure, avec un algorithme de reconstruction, de reconstruire une matrice composée d'éléments d'image, qui représente la distribution des modifications d'impédance dans le plan des électrodes, et pour recevoir des signaux de mesure à plusieurs reprises dans le temps et pour reconstruire des matrices, et avec un dispositif d'affichage, dans lequel l'unité de commande et d'évaluation est conçue en outre pour déterminer une série chronologique de la variation de ventilation globale à partir de la succession des matrices reconstruites comme série chronologique de la modification d'impédance moyenne ou comme une série chronologique d'un volume de respiration mesuré et pour déterminer dans celui-ci une phase d'inspiration ou d'expiration, pour subdiviser la phase d'inspiration ou d'expiration dans la variation de ventilation globale en un nombre (1, 2, ...$M_{étapes}$) d'étapes de modification de volume identique, pour déterminer les instants correspondant à ces étapes de modification de volume identique dans la phase d'inspiration ou d'expiration, pour déterminer pour chaque élément d'image la modification de l'impédance locale entre ces instants et pour déterminer respectivement le rapport de cette modification locale de l'impédance de l'élément d'image sur la modification de volume identique globale, afin de former ainsi une succession locale de modifications d'impédance relatives de l'élément d'image en fonction des étapes de modification de volume identique, **caractérisé en ce que** l'unité de commande et d'évaluation est conçue en outre pour, dans une phase d'inspiration ou d'expiration, pour chaque élément d'image, adapter une droite à la succession locale des modifications d'impédance relatives en fonction du nombre d'étapes (1, 2, ...$M_{étapes}$) des modifications de volume identiques et pour déterminer le point zéro de celle-ci, pour associer à ce point zéro un moment à l'intérieur de la phase d'inspiration ou d'expiration et pour associer à ce moment à l'élément d'image une grandeur de ventilation comme mesure scalaire pour la variation de la modification d'impédance locale et pour afficher l'élément d'image en code de valeur de gris ou de couleur sur le dispositif d'affichage en fonction de la grandeur de ventilation déterminée.

2. Appareil de tomographie par impédance électrique selon la revendication 1, **caractérisé en ce que** l'unité de commande et d'évaluation est conçue en outre pour utiliser comme grandeur de ventilation la série chronologique globale des modifications d'impédance, volume, flux ou pression de respiration provenant d'un appareil respiratoire ou, à partir de ces grandeurs, une pression alvéolaire calculée par l'intermédiaire d'un modèle.

**3.** Appareil de tomographie par impédance électrique selon l'une quelconque des revendications précédentes, **caractérisé en ce que** l'unité de commande et d'évaluation est conçue en outre pour afficher chaque élément d'image par code de valeur de gris ou de couleur sur le dispositif d'affichage, pour les instants déterminés des étapes de modification de volume identique en fonction de la modification d'impédance locale.

**4.** Appareil de tomographie par impédance électrique selon l'une quelconque des revendications 1 à 2, **caractérisé en ce que** l'unité de commande et d'évaluation est conçue en outre pour afficher chaque élément d'image par code de valeur de gris ou de couleur sur le dispositif d'affichage à partir du deuxième parmi la pluralité des instants déterminés, pour les instants déterminés d'étapes de modification de volume identique, en fonction de la différence de la modification d'impédance locale de l'élément d'image et de la modification d'impédance locale à l'instant précédent.

**5.** Appareil de tomographie par impédance électrique selon l'une quelconque des revendications précédentes, **caractérisé en ce que** l'unité de commande et d'évaluation est conçue en outre pour réaliser en temps réel la reconstruction des matrices, la détermination des séries chronologiques et de la mesure scalaire pour la variation de la série chronologique locale pour chaque élément d'image.

**6.** Appareil de tomographie par impédance électrique selon l'une quelconque des revendications 1 à 4, **caractérisé en ce que** l'unité de commande et d'évaluation est conçue en outre pour mettre en mémoire les signaux de mesure et pour réaliser de manière retardée la reconstruction des matrices, la détermination des séries chronologiques et de la mesure scalaire pour la variation de la série chronologique locale pour chaque élément d'image ou pour réaliser la reconstruction des matrices en temps réel et pour mettre en mémoire celle-ci et pour réaliser de manière retardée la détermination des séries chronologiques et de la mesure scalaire pour la variation de la série chronologique locale pour chaque élément d'image.

**7.** Appareil de tomographie par impédance électrique selon l'une quelconque des revendications précédentes, **caractérisé en ce que** l'unité de commande et d'évaluation est conçue en outre pour convertir les mesures scalaires pour la variation des séries chronologiques locales en un code de couleur et pour afficher les éléments d'image de manière codée en couleur sur le dispositif d'affichage.

**8.** Appareil de tomographie par impédance électrique selon l'une quelconque des revendications précédentes, **caractérisé en ce que** l'unité de commande et d'évaluation est conçue en outre pour regrouper les mesures scalaires pour la variation de la modification d'impédance locale dans les éléments d'image en un paramètre global et pour le représenter de manière alphanumérique et/ou graphique sur le dispositif d'affichage.

**9.** Procédé d'enregistrement d'une succession d'images de tomographie par impédance électrique d'un plan en coupe transversale du thorax d'un patient par une pluralité d'électrodes réparties sur la périphérie du thorax, dans lequel, selon le procédé, au moins une paire d'électrodes comme paire d'électrodes d'injection est alimentée avec un courant alternatif ou une tension alternative, respectivement un signal de tension ou signal de courant est enregistré comme signal de mesure avec plusieurs des paires d'électrodes restantes parmi la pluralité des paires d'électrodes et d'autres paires d'électrodes successives parmi la pluralité de paires d'électrodes fonctionnent comme paires d'électrodes d'injection,
une matrice composée d'éléments d'image, qui représente la distribution des modifications d'impédance dans le plan des électrodes, est reconstruite à partir de la totalité des signaux de mesure avec un algorithme de reconstruction,
des matrices de la modification d'impédance sont reconstruites dans le temps à plusieurs reprises,
une série chronologique de la variation de ventilation globale est déterminée à partir de la succession des matrices reconstruites comme série chronologique de la modification d'impédance moyenne ou comme une série chronologique d'un volume de respiration mesuré,
une phase d'inspiration ou d'expiration est déterminée dans la variation de ventilation globale, la phase d'inspiration ou d'expiration subdivisée en un nombre (1, 2, ...$M_{étapes}$) d'étapes de modification de volume identique et les instants correspondant à ces étapes de modification de volume identique dans la phase d'inspiration ou d'expiration sont déterminés,
pour chaque élément d'image, aux instants déterminés de modification de volume identique, le rapport de modification d'impédance de l'élément d'image entre ces instants sur la modification de volume globale identique est formé et ce rapport est déterminé comme succession de la modification d'impédance relative locale en fonction des nombres d'étapes associés de l'élément d'image,
**caractérisé en ce que**

dans une phase d'inspiration ou d'expiration, pour chaque élément d'image, pour adapter une droite à la succession locale des modifications d'impédance relatives en fonction du nombre d'étapes (1, 2, ...$M_{étapes}$) des modifications de volume identiques et le point zéro de celle-ci est déterminé, un moment pendant la phase d'inspiration ou d'expiration est associé à ce point zéro et une grandeur de ventilation est associée à ce moment à l'élément d'image comme mesure scalaire pour la variation de la modification d'impédance locale et l'élément d'image est affiché en code de valeur de gris ou de couleur sur le dispositif d'affichage en fonction de la grandeur de ventilation ainsi déterminée.

FIG. 1

FIG. 2

FIG. 3

EP 2 762 061 B1

pix$=(20,11)$, ITR$_{IOC}=0.26$, pOpen$=15.5$mbar

ITVpOpen$=23.8$ mbar

FIG. 4

EP 2 762 061 B1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1000580 A1

- EP 2228009 A1

**In der Beschreibung aufgeführte Nicht-Patentliteratur**

- Regional Ventilation delay index: detection of tidal recruitment using electric impedance tomography. Yearbook of Intensive Care and Emergency Medicine